(19) Europäisches Patentamt
European Patent Office
Office européen des brevets

(11) **EP 3 321 831 B1**

(12) **EUROPÄISCHE PATENTSCHRIFT**

(45) Veröffentlichungstag und Bekanntmachung des Hinweises auf die Patenterteilung:
**26.06.2019 Patentblatt 2019/26**

(51) Int Cl.:
***G16H 50/50*** *(2018.01)* ***G16H 50/20*** *(2018.01)*

(21) Anmeldenummer: **16198607.0**

(22) Anmeldetag: **14.11.2016**

(54) **VORRICHTUNG ZUM ERMITTELN VON PROGNOSTIZIERTEN SUBJEKTIVEN REFRAKTIONSDATEN ODER PROGNOSTIZIERTEN SUBJEKTIVEN KORREKTIONSDATEN UND COMPUTERPROGRAMM**

DEVICE FOR DETERMINING PREDICTED SUBJECTIVE REFRACTION DATA OR PREDICTED SUBJECTIVE CORRECTION DATA AND COMPUTER PROGRAM

DISPOSITIF DE DÉTERMINATION DE DONNÉES DE RÉFRACTION SUBJECTIVES PRONOSTIQUÉES OU DE DONNÉES DE CORRECTION SUBJECTIVES PRONOSTIQUÉES ET PROGRAMME INFORMATIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**16.05.2018 Patentblatt 2018/20**

(73) Patentinhaber: **Carl Zeiss Vision International GmbH**
**73430 Aalen (DE)**

(72) Erfinder:
• **Ohlendorf, Arne**
**72072 Tübingen (DE)**

• **Wahl, Siegfried**
**73072 Donzdorf (DE)**
• **Leibig, Christian**
**81543 München (DE)**
• **Leube, Alexander**
**72072 Tübingen (DE)**

(74) Vertreter: **PATERIS Patentanwälte PartmbB**
**Markgrafenstrasse 22**
**10117 Berlin (DE)**

(56) Entgegenhaltungen:
**WO-A2-2005/079546 DE-A1-102014 226 824**

**Beschreibung**

Die vorliegende Erfindung betrifft eine Vorrichtung zum Ermitteln von prognostizierten subjektiven Refraktionsdaten oder prognostizierten subjektiven Korrektionswerten für ein Auge auf der Basis von gemessenen objektiven Refraktionsdaten des Auges. Daneben betrifft die Erfindung ein Computerprogramm mit Programmcode, der ein Ermitteln von prognostizierten subjektiven Refraktionsdaten oder prognostizierten subjektiven Korrektionswerten für ein Auge auf der Basis von gemessenen objektiven Refraktionsdaten des Auges ermöglicht.

Die Refraktionsbestimmung betrifft die Bestimmung der Brechungseigenschaften des (menschlichen) Auges. Bei der Refraktionsbestimmung kann zwischen subjektiver Refraktionsbestimmung und objektiver Refraktionsbestimmung unterschieden werden.

Verfahren zur subjektiven Refraktionsbestimmung beruhen dabei auf einer (subjektiven) Rückmeldung einer zu untersuchenden Person über ihre visuelle Wahrnehmung. Ein Beispiel für eine subjektive Refraktionsbestimmung ist eine Bestimmung der Brechungseigenschaften auf Basis von Sehtafeln mit immer kleiner werdender Sehzeichen (z.B. Zahlen oder Buchstaben) oder immer kleiner werdenden Symbolen, wobei die zu untersuchende Person Rückmeldung gibt, welche Zeichen sie erkennen kann.

Herkömmlicherweise erfolgt die subjektive Refraktionsbestimmung mittels Messbrille und Messgläsern oder manuellem oder digitalem Phoropter und unter Verwendung von Sehzeichen, welche auf einem externen Monitor dargestellt werden. Phoropter sind beispielsweise unter https://de.wikipedia.org/wiki/Phoropter, Stand 13.10.2016, beschrieben. Dabei betrachtet eine zu untersuchende Person die Sehzeichen, und ein Augenoptiker oder Augenarzt setzt in die Messbrille verschiedene Messgläser mit verschiedenen Korrektionsstärken ein, oder verändert eine Korrektionseinstellung bei Verwendung eines Phoropters. Die zu untersuchende Person gibt dann eine Rückmeldung, mit welchen Messgläsern bzw. welchen Einstellungen des Phoropters die Sehzeichen am besten erkennbar sind.

Die dabei verwendeten Messgläser bzw. entsprechende Korrektionseinstellungen des Phoropters sind dabei jeweils einem bestimmten Refraktionsfehler zugeordnet, welcher durch das jeweilige Messglas bzw. die Einstellung des Phoropters korrigiert wird. Die vorstehend erläuterte subjektive Refraktionsbestimmung kann dabei getrennt für jedes Auge (monokular) oder auch für beide Augen gemeinsam (binokular) erfolgen.

Verfahren und Vorrichtungen zur objektiven Refraktionsbestimmung benötigen keine Rückmeldung der zu untersuchenden Person über ihre visuelle Wahrnehmung. Es erfolgt eine Messung der Refraktionsfehler unter ggf. rechnerischer Aufbereitung aus den unmittelbar gemessenen Größen. Bspw. beschreibt die WO 2004/112576 A2 ein Verfahren und eine Vorrichtung zum Bestimmen eines Sehschärfemaßes auf der Basis eines gemessenen Wellenfrontfehlers. Das Sehschärfemaß wird dabei unter Verwendung einer Punktbildverwaschungsfunktion (Point Spread Function) aus dem Wellenfrontfehler niederer und höherer Ordnung berechnet. Die objektive Refraktionsbestimmung wird z.B. mit Hilfe von Autorefraktoren, Photorefraktoren, Wellenfrontanalysatoren, Retinoskopen etc. durchgeführt.

Die oben beschriebene Herangehensweise ist im Wesentlichen ortsfest, das heißt sie ist an die Räumlichkeiten des Augenoptikers oder Augenarztes gebunden, und benötigt einen entsprechend geschulten Augenoptiker oder Augenarzt, um die Refraktionsbestimmung durchzuführen. Refraktionsfehler zu bestimmen und gegebenenfalls adäquat zu korrigieren, beispielsweise durch die Verschreibung von Brillen. Eine anschauliche Erklärung der Unterschiede zwischen subjektiver Refraktion, objektiver Refraktion und Verordnung entnimmt man z.B. "https://de.wikipedia.org/wiki/Refraktion_(Augenoptik)", heruntergeladen am 21.10.2016.

Daten, welche die Ametropie (umgangssprachlich Fehlsichtigkeit) eines Auges beschreiben, werden im Rahmen dieser Beschreibung als Refraktionsdaten bezeichnet. Subjektive Refraktionsdaten sind dabei mittels subjektiver Refraktion gewonnene Daten, objektive Refraktionsdaten solche, die mittels objektiver Refraktionsbestimmung ermittelt worden sind. Die subjektiven Refraktionsdaten und/oder die objektiven Refraktionsdaten können bspw. in Form von Zernikekoeffizienten, in der klassischen Notation Sphäre, Zylinder und Achse, wobei Sphäre den sphärischen Brechwert S, Zylinder den zylindrischen Brechwert C und Achse die Lage der Zylinderachse (Achslage) angeben, in der Power-Vektor-Schreibweise (SÄ, $J_0$, $J_{45}$), wobei in SÄ das sog. sphärische Äquivalent, $J_0$ die horizontale oder vertikale Komponente des Kreuzzylinders und $J_{45}$ die zur horizontalen oder vertikalen Komponente unter einem Winkel von 45 ° bzw. 135° verlaufende Komponente des Kreuzzylinders bezeichnen, oder in einer anderen geeigneten Notation angegeben sein. Die Power-Vektoren-Komponenten SÄ, $J_0$ und $J_{45}$ stehen mit den Werten S, C und $\alpha$ für Sphäre, Zylinder und Achse über die Gleichungen

$$S\ddot{A} = S + 0,5 \cdot C$$

$$J_0 = -0,5 \cdot C \cdot \cos(2\alpha)$$

$$J_{45} = -0,5 \cdot C \cdot \sin(2\alpha)$$

in Beziehung.

Korrektionswerte sind Werte, die ein an die jeweils festgestellte Ametropie angepasstes Korrekturmittel wie bspw. ein Brillenglas charakterisieren und die in der klassischen Notation Sphäre, Zylinder und Achse zumindest den Wert S für die Sphäre enthalten. Falls die Ametropie einen Astigmatismus beinhaltet, enthalten die Korrekturwerte auch die Werte C und α für Zylinder und Achse. Darüber hinaus können die Korrekturwerte weitere Werte umfassen, etwa Prisma und dessen Basislage zur Korrektur von Strabismus (Schielen) und einen Wert der Addition (Nahzusatz) enthalten, der die Altersfehlsichtigkeit korrigiert. Die Ergebnisse der objektiven Refraktion und der subjektiven Refraktion können für sich oder in Kombination zur Bestimmung der Korrektionswerte der verordneten Brille (Synonyme: Verordnung, Brillenwerte, Korrektur, Rezeptwerte, Rezept, Verschreibung) verwendet werden. Wenn die mittels der objektiven oder subjektiven Refraktion gewonnenen objektiven oder subjektiven Refraktionsdaten in der Notation Sphäre, Zylinder und Achse vorliegen, können die objektiven oder subjektiven Refraktionsdaten direkt als Korrekturwerte herangezogen werden. Als objektive Korrektionswerte werden im Folgenden Korrektionswerte bezeichnet, die auf objektiven Refraktionsdaten basieren, und als subjektive Korrektionswerte solche, die auf subjektiven Refraktionsdaten basieren.

Seit geraumer Zeit existieren Ansätze, aus der Messung objektiver Refraktionsdaten des Auges Aussagen über die subjektiven Refraktionsdaten oder die subjektiven Korrektionswerte des Auges zu treffen. Dabei können Aberrationen niederer und/oder höherer Ordnung oder einer Kombination daraus verwendet werden.

Aus WO 2013/058725 A1 ist bekannt, dass aus Ergebnissen von objektiven Messungen der Aberrationen des Auges, welche Wellenfrontfehler des Auges charakterisieren, vorläufige subjektive Refraktionsdaten zu prognostizieren sind, die ein Augenoptiker oder ein Augenarzt als Ausgangspunkt für das Ermitteln subjektiver Refraktionsdaten heranziehen kann. Das Ermitteln der vorläufigen subjektiven Refraktionsdaten aus den objektiven Messungen der Aberrationen erfolgt dabei unter Verwendung eines linearen Gleichungssystems mit Koeffizienten, denen Skalierungsfaktoren zugeordnet sind, wobei die für das gemessene Auge geeigneten Werte für die Skalierungsfaktoren mit Hilfe eines Algorithmus ermittelt werden. Mittels des Gleichungssystems und der ermittelten Werte für die Skalierungsfaktoren werden dann die vorläufigen subjektiven Refraktionsdaten berechnet.

In WO 2008/049503 A2 ist ein Verfahren zum Ermitteln einer Brillenglasverschreibung beschrieben, in dem die Brillenglasverschreibung aus objektiven Refraktionsdaten oder eine Kombination aus objektiven Refraktionsdaten und ermittelten subjektiven Refraktionsdaten bestimmt wird.

Aus US 7,357,509 B2 sind Metriken bekannt, welche die subjektiven Auswirkungen einer Wellenfrontaberration des Auges prognostizieren. Die Metriken hängen von der Wellenfrontaberration oder von der Wellenfrontaberration und der neuronalen Transferfunktion ab. Insbesondere beschreibt US 7,357,509 B2 Metriken, in welche die neuronale Kontrastempfindlichkeit eingeht. Die Metriken können zum Bestimmen von prognostizierten Korrektionswerten zur Korrektur einer Ametropie herangezogen werden.

Aus US 2015/0346512 A1 ist ebenfalls ein Verfahren zum Ermitteln einer Brillenglasverschreibung, d.h. zum Ermitteln von Korrektionswerten zur Korrektur einer Ametropie, aus gemessenen Aberrationen des Auges anhand von die Sehqualität repräsentierenden Metriken bekannt. In US 2015/0346512 A1 wird der Metrik ein Term, hinzugefügt, der eine Bewertungsfunktion darstellt, die der Grad an Astigmatismuskorrektion in den prognostizierten Brillenglasverschreibungen berücksichtigt.

Die bisher verwendeten Metriken zur Berechnung von prognostizierten Korrektionswerten zur Korrektur einer Ametropie sind jedoch limitiert in der Berücksichtigung der sinnesphysiologischen Empfindung des Sehens. Meist wird nur eine physiologische Messgröße (die Kontrastempfindlichkeit) berücksichtigt. So wird bspw. in dem in US 7,357,509 B2 beschriebenen Verfahren teilweise die Kontrastempfindlichkeit zur Bestimmung der besten Korrektionswerte mit herangezogen. Die objektiven Refraktionsdaten werden hierbei bspw. mit einem einfachen Autorefraktor oder Wellenfrontaberrometer bestimmt. Eine Metrik aus US 7,357,509 B2 versucht, sowohl den optischen als auch den neuronalen Part der Abbildung zu modellieren, um aus objektiven Refraktionsdaten die bestmöglichen Korrektionswerte zu prognostizieren. Es ist jedoch bekannt, dass vor allem die Kontrastempfindlichkeit sehr starken individuellen Unterschieden unterliegt. Da insbesondere die neuronale Transferfunktion u.U. hochgradig nichtlinear und schwer zu beschreiben ist, sind die erzielten Ergebnisse unter Umständen ungenau. Zudem beschreibt die Berücksichtigung nur der Kontrastempfindlichkeit das Sehen als Kombination aus physikalisch-optischen Eigenschaften und sinnesphysiologischen Empfindungen nicht hinreichend genau sofern keine individuelle CSF (contrast sensitivity function) Verwendung findet.

Die bisherigen Lösungen zur Berechnung prognostizierter subjektiver Refraktionsdaten oder prognostizierter subjektiver Korrektionswerte aus objektiven Messungen der Aberrationen des Auges unter Verwendung von Metriken sind daher entweder langwierig und/oder nicht hinreichend genau im Vergleich zur tatsächlichen Ermittlung der subjektiven Refraktionsdaten mittels eines Phoropters oder einer Messbrille.

Die DE 10 2014 226 824 A1 beschreibt ein Verfahren zum Bereitstellen eines lernbasierten Diagnoseunterstützungsmodells. Dazu werden neue oder erweiterte Trainingsdaten, die in der Regel negative Fallstudien repräsentieren, gesammelt. Dann wird eine Lernsoftware benutzt, um das Diagnosesystem mit Trainingsbeispielen repräsentierende Trainingsdaten zu trainieren. Zur Gestaltung einer Lernsoftware können sogenannte neuronale Netze verwendet werden.

Aus WO 2005/079546 A2 ist eine Vorrichtung zum Ermitteln einer Korrelation zwischen subjektiven Korrektionswerten eines zu untersuchenden Auges und objektiven Refraktionsdaten des zu untersuchenden Auges. Zum Ermitteln der Korrelation kommt ein statistisches Modell zum Einsatz, das mittels eines neuronalen Netzwerks gewonnen worden ist.

Gegenüber der WO 2005/079546 A2 als nächstkommendem Stand der Technik ist es eine Aufgabe der vorliegenden Erfindung, eine vorteilhafte Vorrichtung zum Ermitteln von prognostizierten subjektiven Refraktionsdaten oder prognostizierten subjektiven Korrektionswerten für ein Auge auf der Basis von objektiven Refraktionsdaten zur Verfügung zu stellen, die es ermöglicht, die Zuverlässigkeit der Berechnung der prognostizierten subjektiven Refraktionsdaten bzw. der prognostizierten subjektiven Korrektionswerte aus den objektiven Refraktionsdaten des Auges zu erhöhen.

Es ist eine weitere Aufgabe der vorliegenden Erfindung, ein vorteilhaftes Computerprogrammprodukt mit einer Software zum Ermitteln von prognostizierten subjektiven Refraktionsdaten oder prognostizierten subjektiven Korrektionswerten eines zu untersuchenden Auges auf der Basis von objektiven Refraktionsdaten zur Verfügung zu stellen, das es insbesondere ermöglicht, die Zuverlässigkeit der Berechnung der prognostizierten subjektiven Refraktionsdaten bzw. der prognostizierten subjektiven Korrektionswerte aus den objektiven Refraktionsdaten des Auges zu erhöhen.

Die erste Aufgabe wird durch eine Vorrichtung nach Anspruch 1 gelöst, die zweite Aufgabe durch ein Computerprogrammprodukt nach Anspruch 9. Die abhängigen Ansprüche enthalten vorteilhafte Ausgestaltungen der Erfindung.

Die erfindungsmäße Lösung nutzt Verfahren des maschinellen Lernens. Durch Verwendung eines Trainingsdatensatzes von objektiven Refraktionsdaten mit zugeordneten erfassten subjektiven Refraktionsdaten bzw. zugeordneten erfassten subjektiven Korrektionswerte wird mittels eines Modells hinreichend großer Kapazität und vorzugsweise mit darauf abgestimmter Regularisierung, um Überanpassung (engl. Overfitting) zu vermeiden, eine nichtlineare mehrdimensionale Funktion oder eine Familie von nichtlinearen mehrdimensionalen Funktionen ermittelt, mit deren Hilfe dann aus objektiven Refraktionsdaten subjektive Refraktionsdaten bzw. subjektiven Korrektionswerte prognostiziert werden.

Eine erfindungsgemäße Vorrichtung zum Ermitteln von prognostizierten subjektiven Refraktionsdaten oder prognostizierten subjektiven Korrektionswerten eines zu untersuchenden Auges auf der Basis von objektiven Refraktionsdaten des zu untersuchenden Auges umfasst hierzu eine Auswerteeinheit, welche eine Berechnungseinheit umfasst, die die prognostizierten subjektiven Refraktionsdaten bzw. prognostizierten subjektiven Korrektionswerte des Auges mittels einer Funktion aus den objektiven Refraktionsdaten des Auges berechnet. Die erfindungsgemäße Vorrichtung zeichnet sich dadurch aus, dass die Funktion eine nichtlineare mehrdimensionale Funktion oder eine Familie von nichtlinearen mehrdimensionalen Funktionen ist, welche das Ergebnis des Trainierens eines Regressionsmodells oder Klassifikationsmodells ist, das auf der Basis eines Trainingsdatensatzes, welcher für eine Vielzahl von Probanden jeweils zumindest ermittelte objektive Refraktionsdaten und zugeordnete, durch subjektive Refraktion ermittelte subjektive Refraktionsdaten bzw. zugeordnete, durch subjektive Refraktion ermittelte subjektive Korrektionswerte umfasst, trainiert worden ist. Im Falle des Klassifikationsmodells erhält man diskrete prognostizierte subjektive Refraktionsdaten bzw. diskrete prognostizierte subjektive Korrektionswerte.

Laut Enzyklopädie der Wirtschaftsinformatik - Onine-Lexikon; Heraus. Norbert Gronau, Jörg Becker, Karl Kurbel, Elmar Sinz, Leena Suhl (http://www.enzyklopaedie-der-wirtschaftsinformatik.de), Stand 27.09.2013 ist ein Klassifikationsmodell eine Abbildung, die die Zuordnung von Elementen zu vorgegebenen Klassen beschreibt. Hierbei ergibt sich die Klassenausprägung der diskreten Klassifikationsvariablen aus den Ausprägungen der Attribute der Datenobjekte. Die Grundlage für ein Klassifikationsmodell bildet ein Datenbestand, dessen Datenobjekte jeweils einer vorgegebenen Klasse zugeordnet sind. Ein Klassifikationsmodell wird zur Prognose der Klassenzugehörigkeit von Datenobjekten eingesetzt, deren Klassenzugehörigkeit bislang nicht bekannt ist. Mit einem Regressionsmodell wird eine abhängige, stetige Variable durch mehrere unabhängige Variablen erklärt. Es kann somit ebenfalls zur Prognose des unbekannten Wertes der abhängigen Variablen durch die Ausprägungen der zugehörigen unabhängigen Variablen eingesetzt werden. Der Unterschied zu einem Klassifikationsmodells liegt in der Kardinalität der abhängigen Variablen. Bei einem Klassifikationsmodell liegt eine diskrete, bei einem Regressionsmodell eine stetige Variable vor.

Die Auswerteeinheit kann insbesondere zum Verarbeitung von objektiven Refraktionsdaten in Form von objektiv ermittelten Wellenfrontfehlern, z.B. in Form von objektive Wellenfrontfehler angebenden Koeffizienten eines orthogonalen Funktionensystems, bspw. in Form von Zernikekoeffizienten, ausgelegt sein. Zudem kann die Auswerteeinheit zum Ausgeben von prognostizierten subjektiven Refraktionsdaten bzw. prognostizierten subjektiven Korrektionswerten in Form von Powervektor-Koeffizienten, in der klassischen Notifikation mit Sphäre, Zylinder und Achse oder in jeder anderen geeigneten Notation ausgelegt sein.

Bezüglich des Regressionsmodells kommen alle Ansätze in Frage, welche eine hinreichend hohe Performanz erzielen. Geeignete Ansätze sind beispielsweise lineare Regression auf nichtlinearen Merkmale (polynomielle Features, deep network features, etc.), neuronale Netzwerke, also Netzwerke aus künstlichen Neuronen, mit mehreren Schichten, Support Vector Regression mit nichtlinearen Kernels (z.B. Radial Basis Function Kernel), Entscheidungsbäume, Gauss Prozesse, Ensembling mehrerer Regressoren, etc. Eine Reformulierung des Regressionsmodells als Klassifikationsmodell - durch die Diskretisierung der Ausgabewerte wie zum Beispiel der Sphärenwerte - liefert eine äquivalente Lösung. Davon abgeleitet kommen alle Klassifikationsmethoden, die eine hinreichend hohe Performanz erzielen in Frage. Beispiele für denkbare Klassifizierungsmethoden sind: neuronale Netzwerke, Support Vektor Maschinen, Nächste Nachbarn, Lineare/Quadratische Diskriminantenanalyse, Naive Bayes, Entscheidungsbäume, Gaussprozesse, Ensembling mehrerer Klassifizierer etc. Mit hinreichender Kapazität kann die Abbildung (objektive Refraktion → subjektive Refraktion; sowohl optisch als auch neuronal) erfasst werden, ohne dass das mechanistische Modell für den optischen und neuronalen Part bekannt sein müssen.

Mit der erfindungsgemäßen Vorrichtung kann das Ermitteln prognostizierter subjektiver Refraktionsdaten bzw. prognostizierter subjektiver Korrektionswerte sehr schnell erfolgen, da keine Messung der Kontrastempfindlichkeit erforderlich ist. Zudem ermöglicht es die erfindungsgemäße Vorrichtung, mit der aus dem trainierten Regressionsmodell oder Klassifikationsmodell resultierenden nichtlinearen mehrdimensionalen Funktion bzw. der aus dem trainierten Regressionsmodell oder Klassifikationsmodell resultierenden Familie von nichtlinearen mehrdimensionalen Funktionen aus objektiven Refraktionsdaten eines Patienten zuverlässig subjektive Refraktionsdaten bzw. subjektive Korrektionswerte für den Patienten zu prognostizieren. Das trainierte Modell erfasst dabei alle optischen und neuronalen Beiträge, welche über der von den Trainingsdatensätzen repräsentierten Messpopulation hinweg reproduzierbar sind.

Durch die mit der erfindungsgemäßen Vorrichtung bessere Berücksichtigung von physikalisch-optischen Eigenschaften und sinnesphysiologischen Empfindungen beim Sehen ist es darüber hinaus möglich, spezielle Korrektionswerte für unterschiedliche Sehanforderungen wie zum Beispiel das Sehen bei Nacht oder in der Dämmerung zu prognostizieren. Dies kann zum Beispiel dadurch erfolgen, dass beim Trainieren des Regressionsmodells Trainingsdatensätze Verwendung finden, bei denen die objektiven Refraktionsdaten für eine größere Pupille vorliegen und die durch subjektive Refraktion ermittelten subjektiven Refraktionsdaten bzw. die durch subjektive Refraktion ermittelten subjektiven Korrektionswerte solche subjektiven Refraktionsdaten bzw. subjektiven Korrektionswerte beinhalten, die für eine spezielle Korrektion (z.B. Nachtbedingungen) erforderlich sind. Hierzu kann die Auswerteeinrichtung insbesondere auch Pupillendurchmesserdaten des zu untersuchenden Auges berücksichtigen.

In einer vorteilhaften Ausgestaltung umfasst die Auswerteeinrichtung das Regressionsmodell oder Klassifikationsmodell. Außerdem umfasst sie ein Trainingsmodul, mit dem das Regressionsmodell oder Klassifikationsmodell auf der Basis eines Trainingsdatensatzes, welcher für eine Vielzahl von Probanden jeweils ermittelte objektive Refraktionsdaten und zugeordnete, durch subjektive Refraktion ermittelte subjektive Refraktionsdaten bzw. zugeordnete, durch subjektive Refraktion ermittelte subjektive Korrektionswerte umfasst, zum Erhalt der nichtlinearen mehrdimensionalen Funktion bzw. der Familie von mehrdimensionalen Funktionen trainierbar ist. Insbesondere kann damit die mehrdimensionale Funktion bzw. die Familie von mehrdimensionalen Funktionen bei Bedarf weiter optimiert werden oder an spezielle Anforderungen angepasst werden, wenn die Auswerteeinrichtung eine mit dem Trainingsmodul verbundene Eingangsschnittstelle zum Empfang bzw. Eingeben von Trainingsdatensätzen umfasst. Mit den empfangenen Trainingsdatensätzen kann dann ein weiteres Trainieren des Regressionsmodells erfolgen.

In einer weiteren vorteilhaften Ausgestaltung umfasst die Auswerteeinrichtung eine auf das Regressionsmodell oder Klassifikationsmodell abgestimmte Regularisierungseinheit, um beim Trainieren Überanpassung (Overfitting) zu vermeiden. Einer Überanpassung würde dazu führen, dass die nichtlineare mehrdimensionale Funktion bzw. die Familie von nichtlinearen mehrdimensionalen Funktionen an statistische Schwankungen anstatt an den zugrundeliegenden statistischen Zusammenhang zwischen den objektiven Refraktionsdaten und den durch subjektive Refraktion ermittelten subjektiven Refraktionsdaten bzw. durch subjektive Refraktion ermittelten subjektiven Korrektionswerten angepasst wird. Um dies zu vermeiden, führt die Regularisierungseinheit eine Regularisierung durch, d.h. sie führt zusätzliche Informationen in das Training ein, mit denen eine Anpassung der nichtlinearen mehrdimensionalen Funktion bzw. der Familie von nichtlinearen mehrdimensionalen Funktionen an statistischen Schwankungen unterbunden wird.

Die erfindungsgemäße Vorrichtung kann neben der Auswerteeinrichtung auch eine Refraktionsmessvorrichtung zum Bestimmen und Bereitstellen der objektiven Refraktionsdaten des zu untersuchenden Auges umfassen. Die Refraktionsmessvorrichtung kann dabei eine Ausgabeschnittstelle zum Ausgeben der objektiven Refraktionsdaten aufweisen, die mit einer entsprechenden Eingabeschnittstelle der Auswerteeinheit zum Empfang der objektiven Refraktionsdaten verbunden ist. Die Refraktionsmessvorrichtung kann zum Bestimmen der objektiven Refraktionsdaten auf Verfahren wie Autorefraktion, Photorefraktion, sowie auf Wellenfrontaberrometern oder anderen objektiven Refraktoren basieren.

Im Rahmen der erfindungsgemäßen Vorrichtung kann die nichtlineare mehrdimensionale Funktion bzw. die Familie von nichtlinearen mehrdimensionalen Funktionen auch dazu ausgelegt sein, die prognostizierten subjektiven Refraktionsdaten bzw. prognostizierten subjektiven Korrektionswerte des Auges aus den objektiven Refraktionsdaten des Auges und dem Pupillendurchmesser des Auges zu berechnen, wobei der zum Trainieren verwendeten Trainingsdatensatz für jeden Probanden jeweils auch einen erfassten Pupillendurchmesser umfasst. Es ist dabei auch eine Option, dass die erfindungsgemäße Vorrichtung eine Pupillendurchmessermessvorrichtung zum Bestimmen des Pupillendurchmessers des zu untersuchenden Auges umfasst. Die Pupillendurchmessermessvorrichtung kann dabei zum Erstellen von einen gemessenen Pupillendurchmesser repräsentierenden Pupillendurchmesserdaten ausgebildet sein und eine Ausgabeschnittstelle zum Ausgeben der Pupillendurchmesserdaten aufweisen, die mit einer entsprechenden Eingabeschnittstelle der Auswerteinrichtung zum Empfang der Pupillendurchmesserdaten verbunden ist.

Die erfindungsgemäße Vorrichtung kann insbesondere in ein optisches Beobachtungsgerät integriert sein, das ein Modul zur Refraktionsmessung umfasst. Derartige optische Beobachtungsgeräte können bspw. AutoRefraktoren, Aberrometer, Mikroskope, Operationsmikroskope, Fernrohre, Ferngläser, etc. sein.

Die Erfindung stellt außerdem ein Computerprogrammprodukt mit Programmcode zum Ermitteln von prognostizierten subjektiven Refraktionsdaten oder prognostizierten subjektiven Korrektionswerten eines Auges auf der Basis von objektiven Refraktionsdaten des Auges zur Verfügung. Als Computerprogrammprodukt ist dabei ein auf einem geeigneten Medium gespeicherter und/oder über ein geeignetes Medium abrufbarer Programmcode zu verstehen. Zum Speichern des Programmcodes kann jedes zum Speichern von Software geeignete Medium, beispielsweise eine DVD, ein USB-Stick, eine Flashcard oder dergleichen Verwendung finden. Das Abrufen des Programmcodes kann beispielsweise über das Internet oder ein Intranet erfolgen oder über ein anderes geeignetes drahtloses oder kabelgebundenes Netzwerk.

Der Programmcode des erfindungsgemäßen Computerprogrammprodukts ist derart ausgestaltet, dass das Ermitteln von prognostizierten subjektiven Refraktionsdaten oder prognostizierten subjektiven Korrektionswerten mittels der nachfolgenden Verfahrensschritte erfolgt, wenn er in einem Computer geladen und/oder in einem Computer ausgeführt wird, nämlich:

- Bereitstellen der objektiven Refraktionsdaten;

- Berechnen der prognostizierten subjektiven Refraktionsdaten oder der prognostizierten subjektiven Korrektionswerte auf der Basis einer nichtlinearen mehrdimensionalen Funktion oder einer Familie von nichtlinearen mehrdimensionalen Funktionen, die das Ergebnis des Trainierens eines Regressionsmodells oder Klassifikationsmodells ist, wobei das Regressionsmodell oder Klassifikationsmodell auf der Basis eines Trainingsdatensatzes, welcher für eine Vielzahl von Probanden jeweils zumindest ermittelte objektive Refraktionsdaten und zugeordnete, durch subjektive Refraktion ermittelte subjektive Refraktionsdaten bzw. zugeordnete, durch subjektive Refraktion ermittelte subjektive Korrektionswerte umfasst, trainiert worden ist, und

- Ausgeben der prognostizierten subjektiven Refraktionsdaten oder prognostizierten subjektiven Korrektionswerte des Auges.

Der Programmcode kann dabei das Regressionsmodell oder Klassifikationsmodell und einen optionalen Verfahrensschritt des Trainierens des Regressionsmodells oder Klassifikationsmodells auf der Basis eines Trainingsdatensatzes beinhalten.

Der Programmcode kann insbesondere zum Empfang von objektiven Refraktionsdaten in Form objektiv ermittelter Wellenfrontfehler, z.B. in Form von objektive Wellenfrontfehler angebenen Koeffizienten eines orthogonalen Funktionensystems, etwa. in Form von Zernikekoeffizienten, ausgelegt sein. Zudem kann er zum Ausgeben von prognostizierten subjektiven Refraktionsdaten bzw. prognostizierten subjektiven Korrektionswerten in Form Powervektorkoeffizienten, in der klassischen Notifikation mit Sphäre, Zylinder und Achse oder in jeder anderen geeigneten Notifikation ausgelegt sein.

Um Überanpassung (Overfitting) beim Trainieren des Regressionsmodells zu vermeiden kann der Programmcode zudem eine auf das Regressionsmodell oder Klassifikationsmodell abgestimmte Regularisierung beinhalten. Mit anderen Worten, der Programmcode führt zusätzliche Informationen in das Training ein, die während des Trainings eine Anpassung der nichtlinearen mehrdimensionalen Funktion bzw. der Familie von nichtlinearen mehrdimensionalen Funktionen an statistische Schwankungen anstatt an den zugrundeliegenden statistischen Zusammenhang zwischen den objektiven Refraktionsdaten und den durch subjektive Refraktion ermittelten subjektiven Refraktionsdaten bzw. den durch subjektive Refraktion ermittelten subjektiven Korrektionswerten unterbinden.

Der Programmcode kann so ausgestaltet sein, dass in dem Verfahrensschritt des Berechnens der prognostizierten subjektiven Refraktionsdaten oder der prognostizierten subjektiven Korrektionswerte neben den objektiven Refraktionsdaten des Auges auch der Pupillendurchmesser des Auges berücksichtigt wird. Der zum Trainieren des Regressionsmodells oder Klassifikationsmodells verwendete Trainingsdatensatz umfasst dann für jeden Probanden jeweils auch einen erfassten Pupillendurchmesser.

Mittels des Computerprogrammprodukts kann eine erfindungsgemäße Vorrichtung mit Hilfe eines handelsüblichen Computers realisiert werden. Hinsichtlich der erzielbaren Vorteile wird daher auf die Vorteile der erfindungsgemäßen Vorrichtung verwiesen.

Weitere Merkmale, Eigenschaften und Vorteile der Erfindung ergeben sich aus der nachfolgenden Beschreibung von Ausführungsbeispielen unter Bezugnahme auf die beiliegenden Figuren.

Figur 1     zeigt eine Vorrichtung zum Ermitteln von subjektiven Refraktionsdaten in Form eines Blockschaltbildes.

Figur 2 zeigt eine schematische Darstellung des Ermittelns prognostizierter subjektiver Refraktionsdaten oder prognostizierter subjektiver Korrektionswerte eines zu untersuchenden Auges auf der Basis gemessener objektiver Refraktionsdaten des zu untersuchenden Auges.

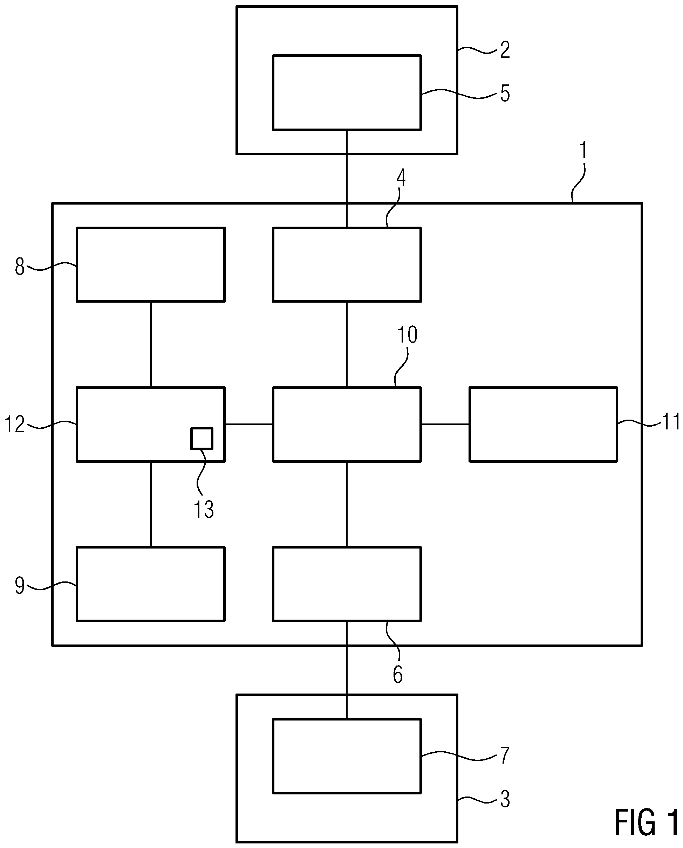

Nachfolgend wird unter Bezugnahme auf Figur 1 ein Ausführungsbeispiel für die erfindungsgemäße Vorrichtung zum Ermitteln von prognostizierten subjektiven Refraktionsdaten oder prognostizierten subjektiven Korrektionswerten eines zu untersuchenden Auges auf der Basis von ermittelten objektiven Refraktionsdaten des zu untersuchenden Auges erläutert. Die Figur zeigt eine Auswerteeinrichtung 1, an die im vorliegenden Ausführungsbeispiel als optionale Ergänzungen eine Refraktionsmessvorrichtung 2 und eine Pupillendurchmessermessvorrichtung 3 angeschlossen sind.

Die Auswerteeinrichtung 1 umfasst eine Eingangsschnittstelle 4 zum Empfangen objektiver Refraktionsdaten, die im vorliegenden Ausführungsbeispiel mit einer Ausgabeschnittstelle 5 der Refraktionsmessvorrichtung 2 verbunden ist. Über die Ausgabeschnittstelle 5 gibt die Refraktionsmessvorrichtung 2 objektive Refraktionsdaten aus, die die an einem Auge gemessenen objektiven Refraktionsdaten repräsentieren.

Zum Ermitteln der objektiven Refraktionsdaten kommt im vorliegenden Ausführungsbeispiel ein Wellenfrontaberrometer zur Anwendung. Alternativ können aber auch Refraktionsmessvorrichtungen Verwendung finden, die auf Autorefraktion oder Fotorefraktion beruhen. Statt von einer Refraktionsmessvorrichtung 2 kann die Eingabeschnittstelle 4 die objektiven Refraktionsdaten aber auch von einem Datenspeicher, etwa einem USB-Stick, einer CD, einer DVD oder einer Speicherkarte, beziehen. Ebenso ist es möglich, die objektiven Refraktionsdaten aus einem Netzwerk zu beziehen bspw. aus dem Internet oder einem Intranet.

Die Auswerteeinrichtung 1 umfasst zudem eine weitere Eingangsschnittstelle 6, die mit einer Ausgangsschnittstelle 7 der Pupillendurchmessermessvorrichtung 3 verbunden ist, die bspw. auch in die Refraktionsmessvorrichtung 2 integriert sein kann. Die Eingangsschnittstelle 6 empfängt Pupillendurchmesserdaten, welche von der Pupillendurchmessermessvorrichtung über die Ausgabeschnittstelle 7 ausgegeben werden und welche den gemessenen Pupillendurchmesser des Auges repräsentieren. Auch hier gilt, dass die Eingabeschnittstelle 6 die Pupillendurchmesserdaten statt von einer Pupillendurchmessermessvorrichtung 3 von einem Datenspeicher, etwa einem USB-Stick, einer CD, einer DVD oder einer Speicherkarte, beziehen kann. Ebenso ist es auch wieder möglich, die Pupillendurchmesserdaten aus einem Netzwerk zu beziehen bspw. aus dem Internet oder einem Intranet.

Außerdem umfasst die Auswerteeinrichtung 1 im vorliegenden Ausführungsbeispiel eine optionale weitere Eingabeschnittstelle 8, über die bei Bedarf Trainingsdatensätze in die Auswerteeinrichtung 1 eingegeben werden können. Die Bedeutung dieser Trainingsdatensätze wird später erläutert werden. Auch die Trainingsdatensätze können von einem Datenspeicher, etwa einem USB-Stick, einer CD, einer DVD oder einer Speicherkarte, oder aus einem Netzwerk, bspw. aus dem Internet oder einem Intranet, bezogen werden.

Als weitere Komponenten umfasst die Auswerteeinrichtung 1 im vorliegenden Ausführungsbeispiel ein Regressionsmodell 9, eine Berechnungseinheit 10, ein Trainingsmodul 12 zum Trainieren des Regressionsmodells 9 und eine Ausgabeschnittstelle 11. Das Trainingsmodul 12 enthält im vorliegenden Ausführungsbeispiel zudem eine Regularisierungseinheit 13, um Überanpassung (Overfitting) während des Trainings des Regressionsmodells 9 zu vermeiden.

Die Berechnungseinheit 10 ist mit der Eingabeschnittstelle zum Empfangen objektiver Refraktionsdaten 4 sowie mit dem Regressionsmodell 9 verbunden. In der Berechnungseinheit 10 werden auf der Basis der empfangenen objektiven Refraktionsdaten die prognostizierten subjektiven Refraktionsdaten bzw. prognostizierten subjektiven Korrektionswerte berechnet. Das Berechnungsergebnis wird dann über die Ausgabeschnittstelle 11 ausgegeben.

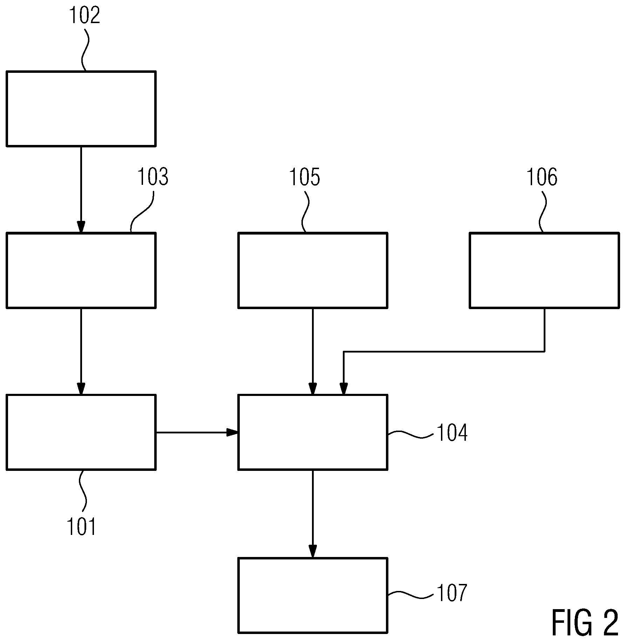

Das Berechnen der prognostizierten subjektiven Refraktionsdaten bzw. der prognostizierten subjektiven Korrektionswerte wird unter Bezugnahme auf das in Figur 2 dargestellte Blockdiagramm beschrieben. Das Berechnen erfolgt im vorliegenden Ausführungsbeispiel auf der Basis einer nichtlinearen mehrdimensionalen Funktion (Block 101), die durch Trainieren des Regressionsmodells 9 gewonnen wird bzw. gewonnen worden ist. Es besteht aber auch die Möglichkeit, das Berechnen auf der Basis einer durch Trainieren eines entsprechenden Regressionsmodells 9 gewonnenen Familie von nichtlinearen mehrdimensionalen Funktionen durchzuführen. Zum Trainieren des Regressionsmodells 9 werden im vorliegenden Ausführungsbeispiel über die Eingabeschnittstelle für Trainingsdatensätze 8 Trainingsdaten eingelesen (Block 102), welche für eine Messpopulation ermittelte objektive Refraktionsdaten und zugeordnete, durch subjektive Refraktion ermittelte subjektive Refraktionsdaten bzw. zugeordnete, durch subjektive Refraktion ermittelte subjektive Korrektionswerte enthalten. Die ermittelten objektiven Refraktionsdaten und die durch subjektive Refraktion ermittelten subjektiven Refraktionsdaten bzw. die durch subjektive Refraktion ermittelten subjektiven Korrektionswerte enthalten dabei für jede Person der Messpopulation einen ermittelten objektiven Refraktionsdatensatz und einen diesem objektiven Refraktionsdatensatz zugeordneten, durch subjektive Refraktion ermittelten subjektiven Refraktionsdatensatz und/oder einen diesem objektiven Refraktionsdatensatz zugeordneten, durch subjektive Refraktion ermittelten subjektiven Korrektionswertesatz.

Im vorliegenden Ausführungsbeispiel liegen die ermittelten objektiven Refraktionsdaten in Form von Zernikekoeffizienten und die ermittelten subjektiven Refraktionsdaten bzw. die ermittelten subjektiven Korrektionswerte in der Notifikation Sphäre, Zylinder und Achse gemäß der DIN EN ISO 13666-213 vor. Zudem enthalten die Trainingsdaten im vorliegenden Ausführungsbeispiel für jede Person der Messpopulation auch Daten über den gemessenen Pupillendurchmesser, welche den objektiven Refraktionsdaten zugeordnet sind. Die Trainingsdaten enthalten also eine in der Regel hohe Anzahl an Trainingsdatensätzen, die jeweils an einem Probanden ermittelte objektive Refraktionsdaten, den ermittelten Pupillendurchmesser sowie die durch subjektive Refraktion ermittelten subjektiven Refraktionsdaten bzw. die durch subjektive Refraktion ermittelten subjektiven Korrektionswerte repräsentieren.

Das Trainieren (Block 103) erfolgt im vorliegenden Ausführungsbeispiel mithilfe eines tiefen neuronalen Netzwerkes (Deep Neural Network (DNN)), mit dessen Hilfe die mittlere quadratische Abweichung zwischen den mit dem Regressionsmodell auf der Basis objektiver Refraktionsdaten prognostizierten subjektiven Refraktionsdaten bzw. prognostizierten subjektiven Korrektionswerte einerseits und den für diese objektiven Refraktionsdaten durch subjektive Refraktion ermittelten subjektiven Refraktionsdaten bzw. durch subjektive Refraktion ermittelten subjektiven Korrektionswerten andererseits minimiert wird. Hierzu wird im vorliegenden Fall ein kleines Multi-Layered Feedforward Network mit einer einzigen linearen Ausgangseinheit verwendet. Das Netzwerk hat zwei versteckte Layer mit 128 bzw. 37 versteckten Einheiten und parametrische Nichtlinearitäten für einen Rectifier, wie dies in He, K. et al., "Delving deep into rectifiers: Surpassing human level performance on ImageNet classification", International Conference on Computer Vision, 2015, http://doi.org/10.1109/ICCV.2015.123 beschrieben ist. Die anfänglichen Gewichte werden von einer Gauß-Funktion mit einem Mittelwert von 0 und einer Standartabweichung von $\sqrt{2/n}$ bezogen, wobei n die Anzahl der eingehenden Verbindungen darstellt. Diese Vorgehensweise ist ebenfalls in He et al. beschrieben. Abwandlungen der Netzwerk-Architektur, d.h. Anzahl der Schichten, Anzahl der Neurone und Typ der Nichtlinearität sind prinzipiell veränderbar. Je nach Stärke der Veränderungen, kann dies Einfluss auf die Performanz (mittlerer quadratischer Fehler, Kostenfunktionen, etc.) haben. Ebenso sind auch alternative Initialisierungen der Netzwerkparameter denkbar. Alle Daten werden durch Abzug des Mittelwertes jeder Merkmalsdimension, die über den Trainingsdatensatz berechnet worden ist, vorbehandelt. Zum Trainieren des Netzwerkes kann bspw. RMSprop mit einer Lernrate von 0,001 verwendet werden, um die mittlere quadratische Abweichung zwischen den prognostizierten subjektiven Refraktionsdaten bzw. Korrektionswerte und den in den Trainingsdatensätzen enthaltenen durch subjektive Refraktion ermittelten subjektiven Refraktionsdaten bzw. Korrektionswerte zu minimieren. Diese Vorgehensweise ist von Hinton et al, "Neutral Networks for Machine Learning, Lecture 6, erhältlich auf "http:// www.cs.toronto.edu/~tijmen/csc321/slides/lecture_slides_lec6.pdf", beschrieben. Um Überanpassung (Overfitting) zu vermeiden, werden im vorliegenden Beispiel kleine Gewichte im Netzwerk mittels L2 Regularisierung mit Lambda = 0,02 bevorzugt.

Als Alternative zum Trainieren mit einem DNN besteht die Möglichkeit, das Trainieren mittels eines elastischen Netzwerkes auf polynomialen Merkmalen (Elastic Net on Polynomial Features) durchzuführen. Hierzu wird beispielsweise eine lineare Regression mit einer Kombination einer L1 (Lasso) und einer L2 (Ridge) Regularisierung (elastisches Netzwerk) auf festgelegten Basisfunktionen durchgeführt. Die Basisfunktionen sind dabei durch eine polynomiale Kombination der Merkmale bis einschließlich der zweiten Ordnung gegeben. Hyperparameter, welche die Gesamtstärke $\alpha$ der Regularisierung und die relative Verteilung $\rho$ von Lasso und Ridge steuern, können über eine Kreuzvalidierung auf dem Trainingsdatensatz eingestellt werden.

Als eine weitere Alternative besteht die Möglichkeit, das Trainieren auf der Basis einer Support Vector Regression mit einem radialen Basisfunktionskern (Support Vector Regression with Radial Basis Function Kernel) durchzuführen. Im Falle der Support Vector Regression kann eine Nichtlinearität dadurch erreicht werden, dass ein radialer Basisfunktionskern Verwendung findet. Die Optimierung der Hyperparameter kann dann mittels Kreuzvalidierung auf den Trainingsdaten durchgeführt werden. Anregungen für Werte von C und $\varepsilon$ enthält Kaneko et al, "Fast Optimization of Hyperparameters for Support Vector Regression Models with Highly Predictive Ability", Chemometrics and Intelligent Laboratory Systems, 142, Seiten 64 bis 69.

Die genannten Methoden wurden getestet, indem eine zehnfache Kreuzvalidierung durchgeführt wurde. Das heißt, für jede Kombination an subjektiven Refraktionsdaten bzw. Korrektionswerte und für jeden der drei beschriebenen Ansätze zum Trainieren wurden 10 Modelle auf der Basis eines Datensatzes getestet, von dem 90 % als Trainingsdaten Verwendung fanden und die verbleibenden 10 % dazu verwendet wurden, die Genauigkeit der Ermittlung der prognostizierten subjektiven Refraktionsdaten bzw. Korrektionswerte aus den objektiven Refraktionsdaten zu überprüfen. Alle drei Verfahren zum maschinellen Lernen und die Überprüfung wurden in *Python 2.7* implementiert. Für den neuronalen Netzwerkteil wurde die Deep Learning Library "keras" verwendet, für das elastische Netzwerk und die Support Vector Regression wurde "scikit-learn" verwendet.

Wieder mit Bezug auf Figur 2 werden also Trainingsdaten eingelesen (Block 102) und mit einem der oben genannten Verfahren, im vorliegenden Ausführungsbeispiel mit DNN, trainiert (Block 103). Das Ergebnis des Trainierens ist dann die mehrdimensionale nichtlineare Funktion (Block 101), die schließlich für das Berechnen der prognostizierten subjektiven Refraktionsdaten bzw. der prognostizierten subjektiven Korrektionswerte aus den objektiven Refraktionsdaten herangezogen wird (Block 104).

Es sei an dieser Stelle angemerkt, dass das Trainieren des Regressionsmodells, um die nichtlineare mehrdimensionale Funktion zu erhalten, vorab durchgeführt werden kann, sodass dieser Schritt beim späteren Berechnen der prognostizierten subjektiven Refraktionsdaten bzw. der prognostizierten subjektiven Korrektionswerte aus den objektiven Refraktionsdaten (Block104) nicht erneut durchgeführt werden muss. Das Berechnen der prognostizierten subjektiven Refraktionsdaten bzw. der prognostizierten subjektiven Korrektionswerte erfordert dann lediglich ein Einlesen (Block 105) der objektiven Refraktionsdaten und, wenn die nichtlineare mehrdimensionale Funktion das Berücksichtigen des Pupillendurchmessers ermöglicht, ein Einlesen der Pupillendurchmesserdaten (Block 106). Da das Trainieren des Regressionsmodells (Block 103) nur einmal vorab zu erfolgen braucht, ist die Geschwindigkeit, mit der das Berechnen der prognostizierten subjektiven Refraktionsdaten bzw. der prognostizierten subjektiven Korrektionswerte aus den objektiven Refraktionsdaten und gegebenenfalls den Pupillendurchmesserdaten (Block 104) erfolgen kann, lediglich durch die Geschwindigkeit, mit der die Berechnung erfolgen kann, und damit durch die Rechenleistung der Vorrichtung begrenzt.

Die berechneten prognostizierten subjektiven Refraktionsdaten bzw. die berechneten prognostizierten subjektiven Korrektionswerte werden schließlich ausgegeben (Block 107). Auf der Basis dieser Daten kann dann ein Rezept für eine Brille, für Kontaktlinsen, für Intraokularlinsen, für die refraktive Chirurgie, etc. erstellt werden. Ebenso besteht die Möglichkeit, auf der Basis der ausgegebenen Daten eine chirurgische Korrektur der Refraktion des Auges vorzunehmen.

Obwohl das Trainieren (Block 103) des Regressionsmodells 9 in der Regel erfolgt, bevor die Vorrichtung zum Ermitteln der prognostizierten subjektiven Refraktionsdaten bzw. der prognostizierten subjektiven Korrektionswerte in der Praxis Verwendung findet, kann es vorteilhaft sein, wenn die Möglichkeit besteht, das Regressionsmodell 9 weiter trainieren zu können. Beispielsweise kann bei Vorhandensein zusätzlicher Trainingsdatensätze die Basis für das Ermitteln der nichtlinearen mehrdimensionalen Funktion verbreitert werden, wodurch die Möglichkeit besteht, die Zuverlässigkeit beim Ermitteln der prognostizierten subjektiven Refraktionsdaten bzw. der prognostizierten subjektiven Korrektionswerte aus den objektiven Refraktionsdaten weiter zu verbessern. Des Weiteren können zusätzliche Datensätze dazu bspw. dazu verwendet werden, regionale Unterschiede oder individuelle Unterschiede in verschiedenen Populationen zu berücksichtigen. Darüber hinaus besteht die Möglichkeit, den Einsatzbereich der Vorrichtung zu erweitern. Wenn die ursprünglichen Trainingsdaten beispielsweise keine Pupillendurchmesserdaten umfasst haben und die trainierte nichtlineare mehrdimensionale Funktion daher nicht in der Lage ist, den Pupillendurchmesser zu berücksichtigen, besteht die Möglichkeit, durch ein erneutes Trainieren mit Trainingsdatensätzen, welche auch Pupillendurchmesserdaten enthalten, erneut zu trainieren, um die Vorrichtung an einen neuen Einsatzbereich anzupassen, beispielsweise an das Ermitteln prognostizierten subjektiver Refraktionsdaten bzw. prognostizierten Korrektionswerte für die Nacht oder die Dämmerung.

Die Auswerteeinrichtung 1 der Vorrichtung zum Ermitteln prognostizierter subjektiver Refraktionsdaten bzw. prognostizierter subjektiver Korrektionswerte kann als ein speziell zu diesem Zweck geschaffenes Gerät realisiert sein. Es besteht aber auch die Möglichkeit, die Auswerteeinheit 1 durch einen handelsüblichen Computer zu realisieren. In diesem Fall sind die Eingangs- und Ausgangsschnittstellen 4, 6, 8, 11 als Datenschnittstellen realisiert und das Regressionsmodell 9 sowie die Berechnungseinheit 10 liegen in Form von Softwaremodulen vor, die durch Programmcode repräsentiert werden können. Der Programmcode kann dann als Computerprogrammprodukt auf einem Speichermedium gespeichert sein oder als Computerprogrammprodukt aus einem Netzwerk abrufbar sein.

Die erfindungsgemäße Vorrichtung zum Ermitteln prognostizierter subjektiver Refraktionsdaten bzw. prognostizierter subjektiver Korrektionswerte auf der Basis objektiver Refraktionsdaten kann in eine optische Vorrichtung, die die objektiven Refraktionswerte misst und dann auf Basis der berechneten prognostizierten subjektiven Refraktionsdaten eine Korrektur vornimmt, integriert werden. Die Korrektur kann dann manuell oder automatisch, einmalig oder wiederholend bzw. kontinuierlich und insbesondere in Echtzeit erfolgen. Eine solche Vorrichtung kann beispielsweise ein Refraktometer, ein Mikroskop, ein Operationsmikroskop, ein Teleskop, ein Fernglas, eine Datenbrille, eine subjektive Refraktionseinheit, etc. sein. Je nach optischem Gerät, in das die Vorrichtung zum Ermitteln prognostizierter subjektiver Refraktionsdaten bzw. prognostizierter subjektiver Korrektionswerte integriert ist, wird entweder die Auswerteeinheit alleine oder die Auswerteeinheit in Kombination mit der Refraktionsmessvorrichtung und/oder der Pupillendurchmessermessvorrichtung in das Gerät integriert. Ist die optische Vorrichtung beispielsweise ein Refraktometer, werden lediglich die Auswerteeinheit und gegebenenfalls die Pupillendurchmessermessvorrichtung in das Refraktometer integriert. Ist die optische Einheit dagegen ein Operationsmikroskop, kann zudem auch noch die Refraktionsmessvorrichtung in das Operationsmikroskop integriert werden. Gleiches gilt für andere optische Geräte, die keine Refraktionsmessvorrichtung beinhalten.

Wenn die erfindungsgemäße Vorrichtung in ein Operationsmikroskop integriert ist, bietet dies die Möglichkeit, intraoperative objektive Messungen am Patienten vorzunehmen und auf der Basis der berechneten prognostizierten subjektiven Refraktionsdaten eine Beurteilung des Operationsergebnisses bzw. der letztendlich erreichten Refraktion vorherzusagen, beispielsweise bei der Implantierung von intraokularen Linsen (Intraokularlinsen, IOLs).

Eine Messung der Refraktion kann insbesondere auch bei mobilen oder transportablen Geräten erfolgen. Hier ist in erster Linie an Smartphones oder Screening Geräte zu denken. Im Falle von Smartphones bietet sich die Implementierung der Erfindung in Form eines Computerprogrammprodukts, nämlich in Form einer App, an, die auf das Smartphone geladen werden kann.

Die vorliegende Erfindung wurde anhand eines Ausführungsbeispiels zu Erläuterungszwecken im Detail beschrieben. Ein Fachmann erkennt jedoch, dass von dem Ausführungsbeispiel abgewichen werden kann. So können beispielsweise statt der Zernike-Koeffizienten auf anderen Polynomen basierende Koeffizienten oder andere Wellenfrontaberrationen kennzeichnende Größen zum Beschreiben der objektiven Refraktionsdaten herangezogen werden. Denkbar ist beispielsweise Koeffizienten zu verwenden, die auf Bessel-Polynomen, Jacobi-Polynomen, Legendre-Polynomen oder anderen orthogonalen Polynomen basieren. Selbst die mathematische Beschreibung der objektiven Refraktionsdaten mittels Splines oder anderen Polynomen sind denkbar. Analog zur alternativen mathematischen Beschreibung der Eingangsdaten können auch alternative Formulierungen des Regressions- bzw. Klassifikationsmodells verwendet werden. Für ein gegebenes konkretes Modell sind insbesondere oft auch in einem gewissen Rahmen alternative Hyperparameter möglich, welche zu äquivalenten Lösungen führen. Die vorliegende Erfindung soll daher nicht auf das beschriebene Ausführungsbeispiel beschränkt sein, sondern lediglich durch die beigefügten Ansprüche.

Bezugszeichenliste

1    Auswerteeinrichtung
2    Refraktionsmessvorrichtung
3    Pupillendurchmessermessvorrichtung
4    Eingangsschnittstelle
5    Ausgabeschnittstelle
6    Eingangsschnittstelle
7    Ausgangsschnittstelle
8    Eingangsschnittstelle
9    Regressionsmodell
10   Berechnungseinheit
11   Ausgabeschnittstelle
12   Trainingsmodul
13   Regularisierungseinheit
101  nichtlineare mehrdimensionale Funktion
102  Einlesen Trainingsdatensätze
103  Trainieren des Regressionsmodells
104  Berechnen der prognostizierten subjektiven Refraktionsdaten bzw. der prognostizierten subjektiven Korrektionswerte
105  Einlesen objektiver Refraktionsdaten
106  Einlesen von Pupillendurchmesserdaten
107  Ausgeben der prognostizierten subjektiven Refraktionsdaten bzw. der prognostizierten subjektiven Korrektionswerte

## Patentansprüche

1.  Vorrichtung zum Ermitteln von prognostizierten subjektiven Refraktionsdaten oder prognostizierten subjektiven Korrektionswerten eines zu untersuchenden Auges auf der Basis von objektiven Refraktionsdaten des zu untersuchenden Auges mit einer Auswerteeinrichtung (1), welche eine Berechnungseinheit (10) umfasst, die die prognostizierten subjektiven Refraktionsdaten bzw. prognostizierten subjektiven Korrektionswerte des Auges mittels einer Funktion (101) aus den objektiven Refraktionsdaten des Auges berechnet, wobei die Funktion das Ergebnis des Trainierens eines Regressionsmodells (9) oder Klassifikationsmodells ist, wobei das Regressionsmodell (9) oder Klassifikationsmodell auf der Basis eines Trainingsdatensatzes, welcher für eine Vielzahl von Probanden jeweils zumindest ermittelte objektive Refraktionsdaten und zugeordnete, durch subjektive Refraktion ermittelte subjektive Refraktionsdaten bzw. zugeordnete, durch subjektive Refraktion ermittelte subjektive Korrektionswerte umfasst, trainiert worden ist (103), **dadurch gekennzeichnet, dass**

    - die Funktion eine nichtlineare mehrdimensionale Funktion (101) oder eine Familie von nichtlinearen mehrdimensionalen Funktionen ist.

2.  Vorrichtung nach Anspruch 1, **dadurch gekennzeichnet, dass** die Auswerteeinrichtung (1) das Regressionsmodell (9) oder Klassifikationsmodell umfasst und außerdem ein Trainingsmodul (12) umfasst, mit dem das Regressionsmodell (9) oder Klassifikationsmodell auf der Basis eines Trainingsdatensatzes, welcher für eine Vielzahl von Probanden jeweils ermittelte objektive Refraktionsdaten und zugeordnete, durch subjektive Refraktion ermittelte subjektive Refraktionsdaten bzw. zugeordnete, durch subjektive Refraktion ermittelte subjektive Korrektionswerte umfasst, zum Erhalt der nichtlineare mehrdimensionalen Funktion (101) bzw. der Familie von nichtlinearen mehrdimensionalen Funktionen trainierbar ist.

3.  Vorrichtung nach Anspruch 2, **dadurch gekennzeichnet, dass** das Trainingsmodul (12) zudem eine auf das Regressionsmodell (9) oder Klassifikationsmodell abgestimmte Regularisierungseinheit (13) umfasst.

**4.** Vorrichtung nach Anspruch 2 oder Anspruch 3, **dadurch gekennzeichnet, dass** sie außerdem eine mit dem Trainingsmodul (12) verbundene Eingangsschnittstelle (8) zum Empfangen bzw. Eingeben von Trainingsdatensätzen umfasst.

**5.** Vorrichtung nach einem der vorangehenden Ansprüche, **dadurch gekennzeichnet, dass** die Auswerteeinrichtung (1) außerdem eine Refraktionsmessvorrichtung (2) zum Bestimmen und Breitstellen der objektiven Refraktionsdaten des zu untersuchenden Auges umfasst.

**6.** Vorrichtung nach einem der vorangehenden Ansprüche, **dadurch gekennzeichnet, dass** die nichtlineare mehrdimensionale Funktion (101) bzw. die Familie von nichtlinearen mehrdimensionalen Funktionen dazu ausgelegt ist, die prognostizierten subjektiven Refraktionsdaten bzw. prognostizierten subjektiven Korrektionswerte des Auges aus den objektiven Refraktionsdaten des Auges und dem Pupillendurchmesser des Auges zu berechnen, wobei der zum Trainieren verwendeten Trainingsdatensatz für jeden Probanden jeweils auch einen erfassten Pupillendurchmesser umfasst.

**7.** Vorrichtung nach Anspruch 6, **dadurch gekennzeichnet, dass** sie außerdem eine Pupillendurchmessermessvorrichtung (3) zum Bestimmen des Pupillendurchmessers des zu untersuchenden Auges umfasst.

**8.** Optisches Beobachtungsgerät mit einer Vorrichtung nach einem der vorangehenden Ansprüche.

**9.** Computerprogrammprodukt mit Programmcode, zum Ermitteln von prognostizierten subjektiven Refraktionsdaten oder prognostizierten subjektiven Korrektionswerten eines Auges auf der Basis von objektiven Refraktionsdaten des Auges mittels der nachfolgenden Verfahrensschritte, wenn der Programmcode in einem Computer geladen und/oder in einem Computer ausgeführt wird, nämlich:

    - Bereitstellen (105) der objektiven Refraktionsdaten (4);
    - Berechnen (104) der prognostizierten subjektiven Refraktionsdaten oder der prognostizierten subjektiven Korrektionswerte auf der Basis einer nichtlinearen mehrdimensionalen Funktion (101) oder einer Familie von nichtlinearen mehrdimensionalen Funktionen, die das Ergebnis des Trainierens eines Regressionsmodells (9) oder Klassifikationsmodells ist, wobei das Regressionsmodell (9) oder Klassifikationsmodell auf der Basis eines Trainingsdatensatzes, welcher für eine Vielzahl von Probanden jeweils zumindest ermittelte objektive Refraktionsdaten und zugeordnete, durch subjektive Refraktion ermittelte subjektive Refraktionsdaten bzw. zugeordnete, durch subjektive Refraktion ermittelte subjektive Korrektionswerte umfasst, trainiert worden ist, und
    - Ausgeben (107) der prognostizierten subjektiven Refraktionsdaten oder prognostizierten subjektiven Korrektionswerte des Auges.

**10.** Computerprogrammprodukt nach Anspruch 9, in dem der Programmcode außerdem einen optionalen Verfahrensschritt des Trainierens (103) des das Regressionsmodells (9) oder Klassifikationsmodells auf der Basis eines Trainingsdatensatzes beinhaltet.

**11.** Computerprogrammprodukt nach Anspruch 10, in dem der Programmcode außerdem Verfahrensschritte zum Durchführen einer auf das Regressionsmodell oder Klassifikationsmodell abgestimmten Regularisierung während des Trainierens (103) umfasst.

**12.** Computerprogrammprodukt nach einem der Ansprüche 9 bis 11, in der Programmcode für den Verfahrensschritt des Berechnens (104) der prognostizierten subjektiven Refraktionsdaten oder der prognostizierten subjektiven Korrektionswerte ein Berechnen der prognostizierten subjektiven Refraktionsdaten oder der prognostizierten subjektiven Korrektionswerte auf der Basis einer nichtlineare mehrdimensionale Funktion (101) oder einer Familie von nichtlinearen mehrdimensionalen Funktionen vorsieht, die neben den objektiven Refraktionsdaten des Auges auch den Pupillendurchmesser des Auges berücksichtigt, wobei der zum Trainieren verwendeten Trainingsdatensatz für jeden Probanden jeweils auch einen erfassten Pupillendurchmesser umfasst.

**Claims**

**1.** Apparatus for ascertaining, on the basis of objective refraction data of an eye to be examined, predicted subjective refraction data or predicted subjective correction values of the eye to be examined, said apparatus comprising an evaluation device (1), which comprises a calculation unit (10) that calculates the predicted subjective refraction data or predicted subjective correction values of the eye by means of a function (101) from the objective refraction data of the eye, wherein the function is the result of training a regression model (9) or classification model, wherein the regression model (9) or classification model has been trained (103) on the basis of a training data record, which, for a multiplicity of subjects, in each case comprises at least ascertained objective refraction data and assigned subjective refraction data ascertained by subjective refraction or assigned subjective correction values ascertained by subjective refraction,
**characterized in that**

    - the function is a nonlinear multidimensional function (101) or a family of nonlinear multidimensional functions.

2. Apparatus according to Claim 1, **characterized in that** the evaluation device (1) comprises the regression model (9) or classification model and moreover comprises a training module (12), by means of which the regression model (9) or classification model is trainable on the basis of a training data record, which, for a multiplicity of subjects, in each case comprises ascertained objective refraction data and assigned subjective refraction data ascertained by subjective refraction or assigned subjective correction values ascertained by subjective refraction, for the purposes of obtaining the nonlinear multidimensional function (101) or the family of nonlinear multidimensional functions.

3. Apparatus according to Claim 2, **characterized in that** the training module (12) moreover comprises a regularization unit (13) that is matched to the regression model (9) or classification model.

4. Apparatus according to Claim 2 or Claim 3, **characterized in that** said apparatus moreover comprises an input interface (8), connected to the training module (12), for receiving or entering training data records.

5. Apparatus according to any one of the preceding claims, **characterized in that** the evaluation device (1) moreover comprises a refraction measuring apparatus (2) for determining and providing the objective refraction data of the eye to be examined.

6. Apparatus according to any one of the preceding claims, **characterized in that** the nonlinear multidimensional function (101) or the family of nonlinear multidimensional functions is configured to calculate the predicted subjective refraction data or predicted subjective correction values of the eye from the objective refraction data of the eye and the pupil diameter of the eye, wherein the training data record used for training purposes in each case also comprises a captured pupil diameter for each subject.

7. Apparatus according to Claim 6, **characterized in that** said apparatus moreover comprises a pupil diameter measuring apparatus (3) for determining the pupil diameter of the eye to be examined.

8. Optical observation appliance having an apparatus according to any one of the preceding claims.

9. Computer program product having program code for ascertaining predicted subjective refraction data or predicted subjective correction values of an eye on the basis of objective refraction data of the eye by means of the subsequent method steps when the program code is loaded onto a computer and/or executed on a computer, specifically:

    - providing (105) the objective refraction data (4) ;
    - calculating (104) the predicted subjective refraction data or the predicted subjective correction values on the basis of a nonlinear multidimensional function (101) or a family of nonlinear multidimensional functions, which is the result of training a regression model (9) or classification model, wherein the regression model (9) or classification model has been trained on the basis of a training data record, which, for a multiplicity of subjects, in each case comprises at least ascertained objective refraction data and assigned subjective refraction data ascertained by subjective refraction or assigned subjective correction values ascertained by subjective refraction, and
    - outputting (107) the predicted subjective refraction data or predicted subjective correction values of the eye.

10. Computer program product according to Claim 9, wherein the program code moreover contains an optional method step of training (103) of the regression model (9) or classification model on the basis of a training data record.

11. Computer program product according to Claim 10, wherein the program code moreover comprises method steps for carrying out a regularization during the training (103), said regularization being matched to the regression model or classification model.

12. Computer program product according to any one of Claims 9 to 11, wherein program code for the method step of calculating (104) the predicted subjective refraction data or the predicted subjective correction values provides for a calculation of the predicted subjective refraction data or the predicted subjective correction values on the basis of a nonlinear multidimensional function (101) or a family of nonlinear multidimensional functions, which also take account of the pupil diameter of the eye in addition to the objective refraction data of the eye, wherein the training data record used for training purposes in each case also comprises a captured pupil diameter for each subject.

## Revendications

1. Dispositif destiné à déterminer des données de réfraction subjectives prédites ou des valeurs de correction subjectives prédites d'un oeil à examiner sur la base de données de réfraction objectives de l'oeil à examiner, le dispositif comprenant un moyen d'évaluation (1), qui comprend une unité de calcul (10) qui calcule les données de réfraction subjectives prédites ou les valeurs de correction subjectives prédites de l'oeil au moyen d'une fonction (101) à partir des données de réfraction objectives de l'oeil, la fonction étant le résultat de l'apprentissage d'un modèle de régression (9) ou d'un modèle de classification, l'apprentissage du modèle de régression (9) ou du modèle de classification ayant été effectué sur la base d'un ensemble de données d'apprentissage qui comprend au moins des données de réfraction objectives déterminées et des données de réfraction subjectives associées, déterminées par réfraction subjective, ou des valeurs de correction subjectives associées, déterminées par réfraction subjective (103), pour une pluralité de sujets testés, **caractérisé en ce que**

   - la fonction est une fonction multidimensionnelle non linéaire (101) ou une famille de fonctions multidimensionnelles non linéaires.

2. Dispositif selon la revendication 1, **caractérisé en ce que** le moyen d'évaluation (1) comprend le modèle de régression (9) ou le modèle de classification et comprend également un module d'apprentissage (12), permettant l'apprentissage du modèle de régression (9) ou du modèle de classification sur la base d'un ensemble de données d'apprentissage qui comprend des données de réfraction objectives déterminées et des données de réfraction subjectives associées, déterminées par réfraction subjective, ou des valeurs de correction subjectives associées, déterminées par réfraction subjective, pour une pluralité de sujets testés, afin d'obtenir la fonction multidimensionnelle non linéaire (101) ou la famille de fonctions multidimensionnelles non linéaires.

3. Dispositif selon la revendication 2, **caractérisé en ce que** le module d'apprentissage (12) comprend également une unité de régularisation (13) adaptée au modèle de régression (9) ou au modèle de classification.

4. Dispositif selon la revendication 2 ou la revendication 3, **caractérisé en ce qu'**il comprend en outre une interface d'entrée (8) reliée au module d'apprentissage (12) et destinée à recevoir ou saisir des ensembles de données d'apprentissage.

5. Dispositif selon l'une des revendications précédentes, **caractérisé en ce que** le moyen d'évaluation (1) comprend en outre un dispositif de mesure de réfraction (2) destiné à déterminer et produire les données de réfraction objectives de l'oeil à examiner.

6. Dispositif selon l'une des revendications précédentes, **caractérisé en ce que** la fonction multidimensionnelle non linéaire (101) ou la famille de fonctions multidimensionnelles non linéaires est conçue pour calculer les données de réfraction subjectives prédites ou les valeurs de correction subjectives prédites de l'oeil à partir des données de réfraction objectives de l'oeil et du diamètre de la pupille de l'oeil, l'ensemble de données d'apprentissage utilisé pour l'apprentissage comprenant pour chaque sujet testé également un diamètre de pupille détecté.

7. Dispositif selon la revendication 6, **caractérisé en ce qu'**il comprend en outre un dispositif de mesure de diamètre de pupille (3) destiné à déterminer le diamètre de pupille de l'oeil à examiner.

8. Appareil d'observation optique comprenant un dispositif selon l'une des revendications précédentes.

9. Progiciel à code de programme destiné à déterminer des données de réfraction subjectives prédites ou des valeurs de correction subjectives prédites d'un oeil sur la base de données de réfraction objectives de l'oeil au moyen des étapes de procédé suivantes, lorsque le code de programme est chargé sur un ordinateur et/ou exécuté sur un ordinateur, à savoir :

   - produire (105) les données de réfraction objectives (4) ;
   - calculer (104) les données de réfraction subjectives prédites ou les valeurs de correction subjectives prédites sur la base d'une fonction multidimensionnelle non linéaire (101) ou d'une famille de fonctions multidimensionnelles non linéaires qui est le résultat de l'apprentissage d'un modèle de régression (9) ou d'un modèle de classification, l'apprentissage du modèle de régression (9) ou du modèle de classification ayant été effectué sur la base d'un ensemble de données d'apprentissage qui comprend au moins des données de réfraction objectives déterminées et des données de réfraction subjectives associées, déterminées par réfraction subjective, ou des valeurs de correction subjectives associées, déterminées par réfraction subjective, pour une pluralité de sujets testés, et
- délivrer (107) des données de réfraction subjectives prédites ou des valeurs de correction subjectives prédites de l'oeil.

10. Progiciel selon la revendication 9, dans lequel le code de programme comprend en outre une étape de procédé facultative de l'apprentissage (103) du modèle de régression (9) ou du modèle de classification sur la base d'un ensemble de données d'apprentissage.

11. Progiciel selon la revendication 10, dans lequel le code de programme comprend en outre des étapes de procédé permettant d'effectuer une régularisation, pendant l'apprentissage (103), adaptée au modèle de régression ou au modèle de classification.

12. Progiciel selon l'une des revendications 9 à 11, dans lequel le code de programme destiné à l'étape de procédé de calcul (104) des données de réfraction subjectives prédites ou des valeurs de correction subjectives prédites prévoit le calcul des données de réfraction subjectives prédites ou des valeurs de correction subjectives prédites sur la base d'une fonction multidimensionnelle non linéaire (101) ou d'une famille de fonctions multidimensionnelles non linéaires qui prend en compte, en plus des données de réfraction objectives de l'oeil, également le diamètre de pupille de l'oeil, l'ensemble de données d'apprentissage utilisé pour l'apprentissage comprenant pour chaque sujet testé également un diamètre de pupille détecté.

FIG 1

FIG 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004112576 A2
- WO 2013058725 A1
- WO 2008049503 A2
- US 7357509 B2
- US 20150346512 A1
- DE 102014226824 A1
- WO 2005079546 A2

**In der Beschreibung aufgeführte Nicht-Patentliteratur**

- **HERAUS. NORBERT GRONAU ; JÖRG BECKER ; KARL KURBEL ; ELMAR SINZ ; LEENA SUHL.** Laut Enzyklopädie der Wirtschaftsinformatik - Onine-Lexikon. 27. September 2013
- **HE, K. et al.** Delving deep into rectifiers: Surpassing human level performance on ImageNet classification. *International Conference on Computer Vision,* 2015
- **HINTON et al.** *Neutral Networks for Machine Learning, Lecture 6, http:// www.cs.toronto.edu/~tijmen/csc321/slides/lecture_slides_lec6.pdf*
- **KANEKO et al.** Fast Optimization of Hyperparameters for Support Vector Regression Models with Highly Predictive Ability. *Chemometrics and Intelligent Laboratory Systems,* vol. 142, 64-69